(19) Europäisches Patentamt
European Patent Office
Office européen des brevets

(11) **EP 3 042 693 B1**

(12) **FASCICULE DE BREVET EUROPEEN**

(45) Date de publication et mention
de la délivrance du brevet:
**13.09.2017 Bulletin 2017/37**

(51) Int Cl.:
***A61N 1/37*** *(2006.01)*

(21) Numéro de dépôt: **16150461.8**

(22) Date de dépôt: **07.01.2016**

(54) **DISPOSITIF MÉDICAL IMPLANTABLE ACTIF, NOTAMMENT DE TYPE CAPSULE AUTONOME, À OPTIMISATION DYNAMIQUE DE L'ENERGIE DES IMPULSIONS DE STIMULATION**

AKTIVE IMPLANTIERBARE MEDIZINISCHE VORRICHTUNG, INSBESONDERE VOM TYP AUTONOME KAPSEL, MIT DYNAMISCHER OPTIMIERUNG DER ENERGIE DER STIMULATIONSIMPULSE

ACTIVE IMPLANTABLE MEDICAL DEVICE, SUCH AS AN AUTONOMOUS CAPSULE, WITH DYNAMIC OPTIMISATION OF THE ENERGY OF STIMULATION PULSES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **12.01.2015 FR 1550203**

(43) Date de publication de la demande:
**13.07.2016 Bulletin 2016/28**

(73) Titulaire: **Sorin CRM SAS**
**92140 Clamart Cedex (FR)**

(72) Inventeur: **MAKDISSI, Alaa**
**75011 PARIS (FR)**

(74) Mandataire: **Grünecker Patent- und Rechtsanwälte**
**PartG mbB**
**Leopoldstraße 4**
**80802 München (DE)**

(56) Documents cités:
**WO-A1-94/12237     WO-A1-95/34343**
**US-A- 5 697 956     US-A1- 2004 064 162**
**US-B1- 6 738 668**

EP 3 042 693 B1

**Description**

L'invention concerne les "dispositifs médicaux implantables actifs" tels que définis par la directive 90/385/CEE du 20 juin 1990 du Conseil des communautés européennes, plus précisément les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

Elle concerne notamment, mais de manière non limitative, ceux de ces dispositifs qui se présentent sous la forme d'une capsule autonome destinée à être implantée dans une cavité cardiaque, notamment dans le ventricule. Ces capsules sont dépourvues de toute liaison mécanique et électrique à un dispositif principal distant, implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless*", pour les distinguer des électrodes ou des capteurs disposés à l'extrémité distale d'une sonde (*lead*) conventionnelle, parcourue sur toute sa longueur par un ou plusieurs conducteurs reliant par voie galvanique l'électrode ou le capteur à un générateur connecté à une extrémité opposée, proximale, de la sonde. Une électrode de détection/stimulation en contact avec la paroi du ventricule permet de détecter la présence ou non d'une onde de dépolarisation spontanée de la cavité cardiaque, ainsi que l'instant de survenue de cette onde (marqueur ventriculaire ou auriculaire). L'électrode permet également de délivrer une impulsion de stimulation en cas de dépolarisation spontanée absente ou tardive, de manière à provoquer la contraction de la cavité cardiaque.

On notera toutefois que le caractère autonome de la capsule n'est pas en soi une caractéristique nécessaire de la présente invention.

La gestion de l'énergie de stimulation est un aspect critique de tout stimulateur implanté, car il a une incidence directe sur la consommation électrique de la pile intégrée à ce stimulateur, et donc sur sa durée de vie globale.

Ce sujet est particulièrement critique dans le cas d'un stimulateur de type capsule *leadless* où, contrairement aux stimulateurs conventionnels, l'énergie nécessaire à la délivrance de la stimulation représente près de 70 % de l'énergie totale consommée. De plus, il faut tenir compte de ce que les très faibles dimensions d'une capsule *leadless* imposent de réduire à un minimum la taille de la pile et donc sa capacité, et que dans une capsule *leadless* la pile occupe souvent plus de 70 % du volume du dispositif.

De fait, si l'on pouvait réduire par exemple de moitié l'énergie nécessaire à la stimulation, on pourrait réduire corrélativement d'environ 40 % la taille de la pile en gardant la même longévité, ce qui permettrait de réduire le volume de la capsule à environ 0,6 cm$^3$ (contre 1 cm$^3$ dans le meilleur des cas actuellement), toutes performances égales par ailleurs.

Pour réduire le plus possible l'énergie dédiée à la stimulation en conservant l'efficacité des impulsions électriques délivrées, on peut employer une technique dite de "capture cycle-à-cycle", qui consiste à maintenir l'énergie de stimulation à un niveau minimal en vérifiant en permanence, après chaque stimulation, si celle-ci a été efficace ("capture") ou non. Si aucune onde de dépolarisation n'a été induite par la stimulation de la cavité cardiaque ("absence de capture"), l'implant va délivrer au cours du même cycle cardiaque une stimulation d'une énergie relativement élevée afin de garantir le déclenchement d'une dépolarisation. Ensuite, par itérations successives, l'énergie de stimulation est progressivement réduite à chaque cycle cardiaque afin de converger à nouveau vers une énergie proche de la limite du "seuil d'entrainement" nécessaire pour provoquer la dépolarisation de la cavité cardiaque.

L'invention concerne plus précisément cette manière de déterminer par approches successives le seuil d'entrainement, de la façon la plus économe possible du point de vue de la consommation énergétique.

La technique de base, qui est celle couramment utilisée jusqu'à aujourd'hui dans la majorité des stimulateurs, est celle décrite dans le brevet US 3 777 762 A, en procédant par diminutions progressives de l'amplitude (tension) des impulsions de stimulation, pour une largeur d'impulsions fixe.

Une autre technique est décrite dans le brevet US 4 979 507 A, pour tenir compte du fait que l'énergie délivrée dépend non seulement de l'amplitude des impulsions de stimulation, mais également de la largeur de ces impulsions (durée de la stimulation), le seuil d'entrainement variant en fonction de ces deux paramètres selon une loi non linéaire dite "loi de Lapicque".

La technique proposée dans ce document consiste à effectuer deux balayages en tension, à deux largeurs d'impulsions différentes. Cette manière de procéder présente un risque de défaut de capture, car la loi théorique de Lapicque définit une frontière entre capture et non-capture qui, concrètement, varie d'un patient à l'autre. Il est donc nécessaire de valider en permanence ou à intervalles réguliers la manière dont cette loi s'applique pour chaque patient, en faisant un balayage complet de toutes les valeurs possibles des deux paramètres (amplitude et largeur de l'impulsion de stimulation). Or, un balayage complet est difficilement réalisable, car il est très coûteux en termes d'énergie et nécessite d'interrompre la thérapie pendant le balayage.

Le WO 94/12237 A1 décrit une autre technique d'ajustement automatique du seuil de capture dans laquelle, ici encore, la variation de l'énergie de stimulation d'une impulsion à la suivante est opérée soit par changement de la durée de l'impulsion de stimulation, soit par changement de l'amplitude de cette impulsion. Ceci augmente de façon importante le nombre d'itérations nécessaires à l'algorithme de recherche pour déterminer la valeur effective du seuil d'entrainement.

Les brevets US 5 718 720 A, US 5 702 427 A, US 5 549 652 A et US 6 650 940 B1 décrivent d'autres techniques encore de détermination du seuil d'entrainement, mettant en oeuvre divers modes de détection de la capture tels que : détection directe de la contraction mécanique du myocarde, analyse d'un signal accélérométrique, d'un signal de température, de pression intracardiaque, etc.

Le but de l'invention est de proposer une nouvelle technique de recherche d'un optimum des deux paramètres définissant l'énergie délivrée par l'impulsion de stimulation, à savoir la tension de stimulation (c'est-à-dire l'amplitude de l'impulsion), et la durée de la stimulation (c'est-à-dire la largeur de l'impulsion), de la manière à la fois la plus rapide et la plus économe en termes de consommation énergétique.

Le problème à résoudre est de minimiser le nombre de stimulations à délivrer pour déterminer le seuil d'entrainement, de manière à réduire par voie de conséquence la consommation électrique de l'implant afin d'en améliorer l'autonomie globale et la durée de vie.

Le point de départ de l'invention consiste, contrairement aux techniques de recherche connues qui opèrent généralement par balayage de valeurs successives d'amplitude pour une largeur d'impulsion donnée, à exécuter un algorithme de recherche simultanément dans deux dimensions (amplitude et largeur d'impulsion), c'est-à-dire avec possibilité de faire varier l'un et l'autre des deux paramètres d'une impulsion à la suivante selon un mécanisme fonction du résultat (présence ou absence de capture) de la stimulation précédente.

Comme on le verra également, l'invention propose un tel algorithme opérant de manière itérative par dichotomie, sur la base d'une minimisation de l'énergie totale de l'impulsion, et non plus de la seule minimisation de la tension de l'impulsion.

Plus précisément, l'invention propose un dispositif médical implantable actif comprenant, de manière en elle-même connue :

- un circuit de stimulation ventriculaire, apte à délivrer des impulsions de stimulation de faible énergie à une électrode implantable dans une cavité cardiaque d'un patient ;
- un circuit de test de capture, apte à détecter au cours d'un cycle cardiaque la présence ou l'absence d'une contraction consécutive à l'application d'une impulsion de stimulation ; et
- un circuit d'ajustement de l'énergie des impulsions délivrées par le circuit de stimulation, apte à contrôler indépendamment la tension de stimulation et la largeur de l'impulsion de stimulation.

De façon caractéristique de l'invention, le circuit d'ajustement d'énergie comprend des moyens mettant en oeuvre un algorithme itératif de recherche d'optimum énergétique apte à modifier à la fois la largeur d'impulsion $t$ et la tension $V$ de chaque nouvelle impulsion délivrée, ces moyens étant aptes, à chaque itération courante, à effectuer les actions suivantes :

- définition d'une valeur $\{t, V\}$ d'énergie haute,
- définition d'une valeur $\{t', V\}$ d'énergie basse, avec $t' < t$ et $V' < V,$
- délivrance d'une impulsion de stimulation avec la valeur d'énergie basse puis test de capture, et

  • en présence d'une capture, fin de l'itération courante et passage à une nouvelle itération, avec l'énergie basse courante comme nouvelle valeur d'énergie haute,
  • en l'absence de capture, i) application d'une impulsion consécutive de contre-stimulation de largeur d'impulsion $t$ et de tension $V$ définies pour ladite valeur d'énergie haute, ii) achèvement de l'algorithme et iii) sélection de la dernière valeur d'énergie ayant produit la capture comme valeur d'optimum énergétique.

Dans un mode de réalisation préférentiel, les moyens mettant en oeuvre un algorithme itératif sont également aptes, à chaque itération courante, à effectuer les actions suivantes :

- définition d'une première valeur d'énergie intermédiaire $\{t', V\}$,
- définition d'une deuxième valeur d'énergie intermédiaire $\{t, V'\}$,
- définition d'une troisième valeur d'énergie intermédiaire $\{t'', V''\}$, avec $t'<t''<t$ et $V'<V''<V,$
- classement des première, deuxième et troisième valeurs d'énergie intermédiaires par valeur d'énergie décroissante,

et, en l'absence de capture après délivrance de l'impulsion avec la valeur d'énergie basse puis test de capture :

- poursuite de l'itération courante avec délivrance d'impulsions de stimulation successivement avec les première, deuxième et troisième valeurs d'énergie intermédiaires classées par valeur d'énergie décroissante, jusqu'à détecter une capture, et

e n présence d'une capture, fin de l'itération courante et passage à une nouvelle itération, avec l'énergie intermédiaire courante ayant produit la capture comme nouvelle valeur d'énergie haute, et

e n l'absence de capture, achèvement de l'algorithme et sélection de la dernière valeur d'énergie ayant produit la capture, parmi les première, deuxième et troisième valeurs d'énergie intermédiaire, comme valeur d'optimum énergétique.

La troisième valeur d'énergie intermédiaire peut notamment être une valeur $\{t'', V''\}$ telle que $t'' = (t+t')/2$ et $V'' = (V+V')/2$.

Selon diverses caractéristiques subsidiaires avantageuses :

- les valeurs d'énergie des impulsions délivrées par le circuit de stimulation sont au plus égales à une valeur limite maximale d'énergie, et dans lequel la valeur d'énergie haute $\{t, V\}$ de la première itération de l'algorithme est ladite valeur limite maximale d'énergie ;
- les valeurs d'énergie des impulsions délivrées par le circuit de stimulation sont au moins égales à une valeur limite minimale d'énergie $\{t_L, V_L\}$ (L), et dans lequel ladite valeur d'énergie basse est une valeur $\{t', V'\}$ telle que $t' = (t+t_L)/2$ et $V'=(V+V_L)/2$ ;
- les valeurs d'énergie des impulsions délivrées par le circuit de stimulation sont comprises entre une valeur limite maximale d'énergie et une valeur limite minimale d'énergie calculées avant chaque première itération de l'algorithme ;
- dans ce dernier cas, la largeur d'impulsion et la tension de la valeur limite maximale et de la valeur minimale d'énergie sont calculées par application de facteurs multiplicateurs, respectivement supérieur et inférieur à l'unité, à la largeur d'impulsion courante et à la tension courante du circuit de stimulation avant la première itération de l'algorithme.

On va maintenant décrire un exemple de mise en oeuvre de la présente invention, en référence aux dessins annexés où les mêmes références désignent d'une figure à l'autre des éléments identiques ou fonctionnellement semblables.

La Figure 1 est une vue perspective d'ensemble d'une capsule *leadless.*
La Figure 2 est une vue en coupe longitudinale de la capsule *leadless* de

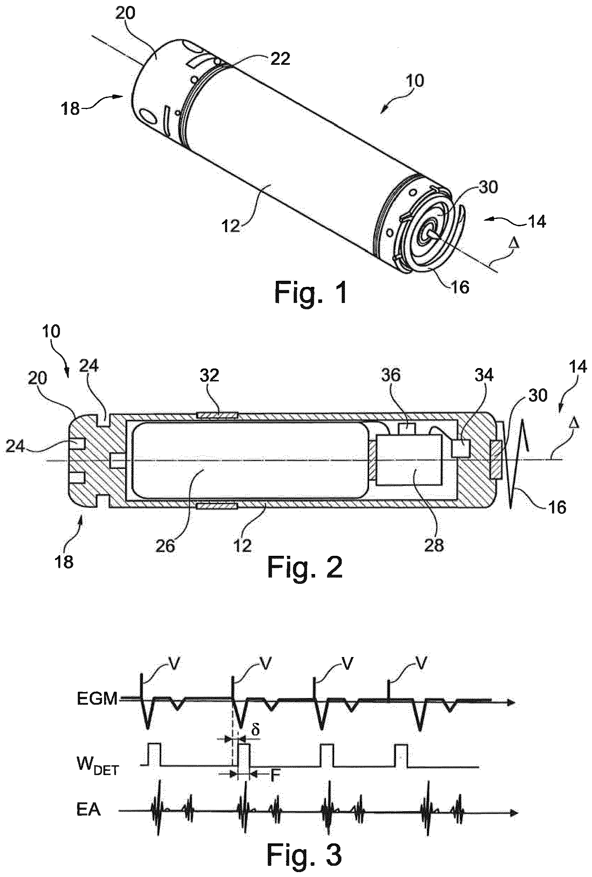

la Figure 1, montrant les principaux composants internes qui la constituent.
La Figure 3 est une série de chronogrammes illustrant, en regard, un signal d'électrogramme EGM, les fenêtres d'analyse pour le test de capture, et le signal d'accélération endocardiaque EA.

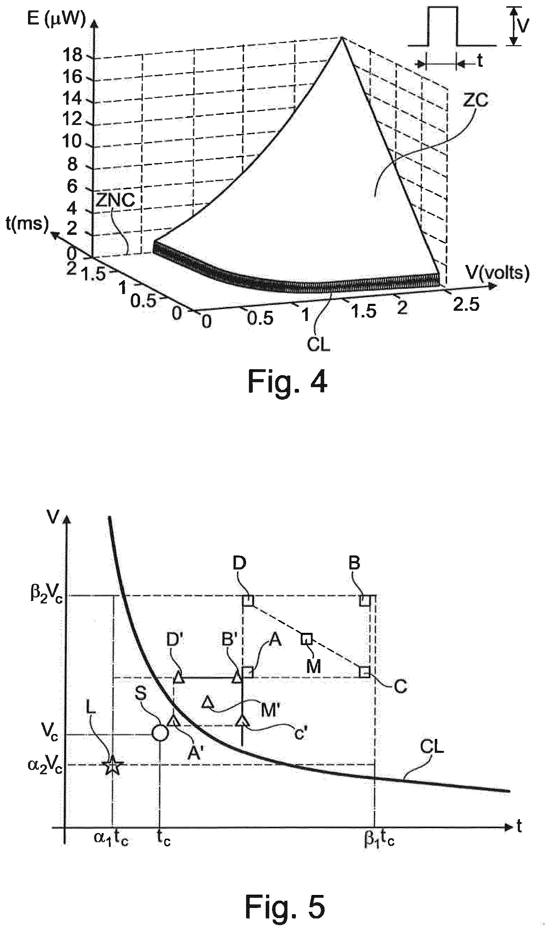

La Figure 4 est une représentation en trois dimensions de l'énergie dépensée par l'application d'une impulsion de stimulation, en fonction de l'amplitude et de largeur de cette impulsion.
La Figure 5 est une représentation bidimensionnelle, en fonction de l'amplitude et de la largeur des impulsions de stimulation successives, explicitant la technique de recherche par dichotomie selon l'invention, avec à chaque itération modification, concurremment, de l'amplitude et de la largeur de l'impulsion délivrée.

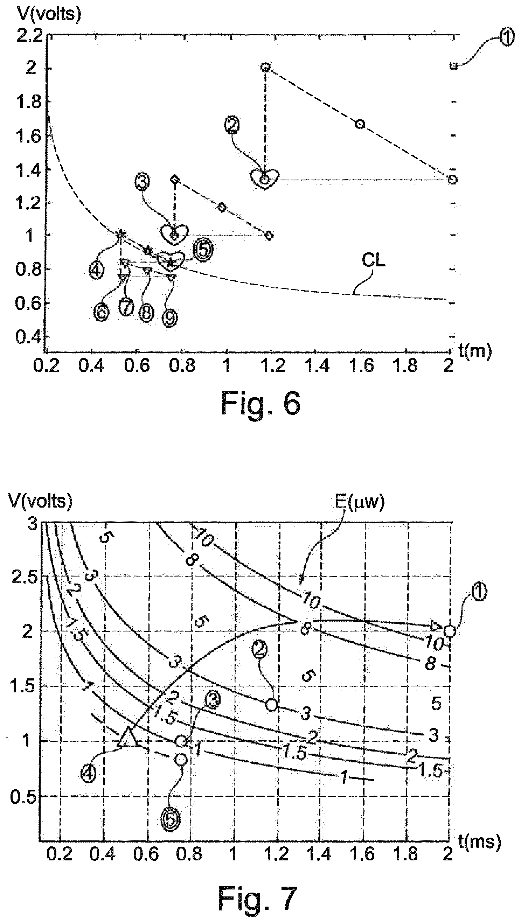

La Figure 6 est une représentation du même type que celle de la Figure 5, appliquée à un premier exemple illustratif de mise en oeuvre de l'algorithme de la Figure 5.
La Figure 7 est un diagramme tension/largeur d'impulsion correspondant à l'exemple de la Figure 6, sur lequel ont été ajoutées les courbes isoénergétiques.

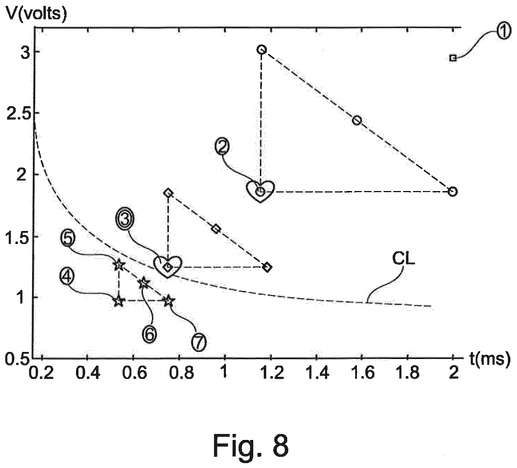

La Figure 8 est homologue de la Figure 6, pour une application à un second exemple illustratif de mise en oeuvre de l'invention.

On va maintenant décrire un exemple de réalisation du dispositif de l'invention.
En ce qui concerne ses aspects logiciels, l'invention peut être mise en oeuvre par une programmation appropriée du logiciel de commande d'un dispositif stimulateur cardiaque connu, par exemple un stimulateur de type capsule *leadless* endocavitaire.
Ces dispositifs comprennent un microprocesseur programmable couplé à des circuits pour recevoir, mettre en forme et traiter des signaux électriques recueillis par des électrodes implantées, et délivrer des impulsions de stimulation à ces électrodes. L'adaptation de ces dispositifs pour réaliser l'invention est à la portée de l'homme du métier, et elle ne sera pas décrite en détail. En particulier, les logiciels conservés en mémoire et exécutés pourront être adaptés et utilisés pour mettre en oeuvre les fonctions de l'invention qui seront décrites ci-dessous
Le procédé de l'invention est en effet mis en oeuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts et/ou de circuits interconnectés, mais cette représentation n'a toutefois qu'un caractère illustratif, ces fonctions ou circuits comprenant des éléments communs et correspondant en pratique à une pluralité de fonctions globalement exécutées par un même logiciel.

Les Figures 1 et 2 montrent, respectivement en perspective et en coupe longitudinale, un exemple de capsule *leadless*, avec les différents organes qui la constituent.

Sur ces figures, la référence 10 désigne de façon générale la capsule, réalisée sous forme d'un corps tubulaire cylindrique 12 d'axe Δ enfermant les divers circuits électroniques et d'alimentation de la capsule. Les dimensions typiques d'une telle capsule sont un diamètre de l'ordre de 6 mm environ pour une longueur d'environ 25 mm.

À son extrémité distale 14, la capsule porte une vis d'ancrage hélicoïdale 16 permettant sa fixation dans les tissus, par exemple contre une paroi d'une cavité cardiaque. Cette vis peut éventuellement être une vis active, électriquement conductrice, pour le recueil de potentiels de dépolarisation cardiaque et/ou l'application d'impulsions de stimulation. La région proximale 18 de la capsule 10 présente une extrémité arrondie, atraumatique 20 et elle est pourvue de moyens de préhension 22, 24 utilisables pour l'implantation ou l'explantation de la capsule.

Comme illustré Figure 2, la capsule 10 incorpore une pile 26 typiquement avec une densité volumétrique d'énergie de l'ordre de 0,8 à 2 W.h/cm$^3$, un module électronique 28, une électrode frontale 30, et éventuellement une électrode latérale 32. Des traversées telles que 34 permettent de relier les électrodes au module électronique 28.

Le module électronique 28 inclut l'ensemble de l'électronique permettant de piloter les diverses fonctions de l'implant, la mémorisation des signaux recueillis, etc. Il comprend un microcontrôleur et un oscillateur générant les signaux d'horloge nécessaires au fonctionnement du microcontrôleur et à la communication. Il contient également un convertisseur analogique/numérique et une mémoire de stockage numérique. Il peut également contenir des moyens émetteurs/récepteurs pour l'échange d'informations avec d'autres dispositifs implantés par communication de type HBC (*Human Body Communication*, communication par voie intracorpo-relle).

La capsule intègre par ailleurs un capteur 36 d'accélération endocardiaque (EA) susceptible de délivrer un signal représentatif de l'activité mécanique du myocarde, par exemple un capteur en forme de microaccé-léromètre interfacé avec le module électronique 28.

Le capteur 36 de signal EA peut être un capteur accélérométrique 1D, 2D ou 3D. De préférence, le capteur est un capteur de type piézoélectrique ou capacitif, mais d'autres types de capteur (optique, résistif, inductif...) capables de générer un signal corrélé au déplacement, à la vitesse ou à l'accélération des parois cardiaques peuvent être utilisés.

La Figure 3 montre une série de chronogrammes illustrant, en regard, un signal d'électrogramme EGM, des fenêtres d'analyse $W_{DET}$ pour le test de capture, et le signal d'accélération endocardiaque EA.

Après chaque stimulation (marqueur V de dépolarisation stimulée sur l'électrogramme EGM), la mesure du signal EA délivré par l'accéléromètre intégré est activée pendant une fenêtre $W_{DET}$ qui est ouverte soit immédiatement après la délivrance de l'impulsion de stimulation, soit avec un retard δ de l'ordre de 5 à 100 ms. La durée F de la fenêtre $W_{DET}$ est comprise entre 75 et 350 ms. Le contrôle de l'instant de début de la fenêtre de capture $W_{DET}$ et de sa durée est réalisé au moyen du circuit de séquencement du microcontrôleur et du logiciel embarqué qui pilote les circuits électroniques de l'implant.

La demande EP 2 412 401 A1 (Sorin CRM) décrit une technique de test de capture par analyse d'un signal EA, notamment des composantes successives (composantes EA) de ce signal qui correspondent aux bruits majeurs du coeur qu'il est possible de reconnaitre dans chaque cycle cardiaque (sons S1 et S2 du phonocardiogramme). Les variations d'amplitude de la première de ces composantes (composante EA1) sont étroitement liées aux variations de la pression dans le ventricule, tandis que la seconde composante (composante EA2) survient pendant la phase de relaxation ventriculaire isovolumique. L'analyse peut également prendre en compte la composante secondaire (composante dite EA4 ou EA0) produite par la contraction de l'oreillette.

Ces composantes sont analysées pour en extraire divers paramètres significatifs tels que l'amplitude crête-à-crête des pics PEA1 et PEA2 des composantes EA1 et EA2, l'intervalle temporel entre ces pics PEA1 et PEA2, la largeur à mi-hauteur des composantes EA1 et/ou EA2, les instants de début et de fin de ces composantes, etc. Il peut également s'agir de paramètres morphologiques représentatifs de la forme d'onde du signal EA ou de son enveloppe.

Cette technique de capture par analyse d'un signal EA n'est toutefois pas limitative de l'invention et l'on peut par exemple procéder, comme décrit par exemple dans la demande EP 0 552 357 A1 (ELA Médical), par analyse des signaux EGM de dépolarisation du myocarde afin de reconnaitre la présence ou non d'une onde évoquée consécutive à l'application de l'impulsion de stimulation.

Le concept de base de l'invention consiste, à la différence des techniques connues qui opèrent souvent un balayage de l'amplitude de l'impulsion de stimulation à largeur d'impulsion constante, à opérer un algorithme de recherche simultanément dans les deux dimensions, amplitude et largeur d'impulsion.

L'énergie dépensée par la délivrance d'une impulsion de stimulation d'amplitude (tension) Vet de largeur *t* est donnée par :

$$E(V,t) = \frac{V^2 t}{R}$$

R étant l'impédance des tissus cardiaques entre les deux électrodes de stimulation.

La Figure 4 montre la manière dont varie l'énergie *E* dépensée par une impulsion de stimulation en fonction des deux paramètres *V* et *t*. Cette représentation comprend deux domaines, avec une zone de capture ZC où l'énergie délivrée a été suffisante pour provoquer une contraction du myocarde, et une zone de non-capture ZNC où cette énergie de stimulation n'a pas été suffisante pour provoquer la contraction. Ces deux zones sont séparées par une frontière CL, correspondant à la courbe théorique de Lapicque, qui est une frontière théorique non linéaire, pouvant varier d'un patient à l'autre. Dans la zone de capture ZC, l'énergie de stimulation augmente avec la tension et avec la largeur de l'impulsion, selon une loi non linéaire.

L'énergie *E* (*V, t*) est l'énergie effectivement dissipée dans l'impédance *R*, c'est-à-dire dans les tissus cardiaques. L'énergie réellement consommée par la source d'énergie électrique de l'implant (batterie ou pile rechargeable) est égale à :

$$E_p(V,t) = \frac{V^2 t}{\eta(V)\,R}$$

où $\eta(V)$ est le rendement du circuit de génération de la tension de stimulation V.

On va maintenant exposer, en référence à la Figure 5, la technique de recherche de l'optimum énergétique par dichotomie selon l'invention.

Il s'agit d'atteindre en un minimum d'étapes les conditions de stimulation (amplitude et largeur de l'impulsion) qui minimisent l'énergie nécessaire à la délivrance d'impulsions procurant une capture effective.

On supposera que le circuit de stimulation est ajusté, à un instant donné, avec des paramètres de stimulation courants $t_c$ et $V_c$ correspondant au point S de coordonnées {$t_c$, $V_c$}.

Le point L représente la valeur d'énergie de stimulation minimale à tester au cours de la phase de recherche, ce point étant avantageusement défini en fonction du point S (c'est-à-dire que la position de L n'est pas fixe mais dépend de l'énergie de stimulation courante) :

$$\vec{L} = (\alpha_1 t_c,\, \alpha_2 V_c)$$

où $\alpha_1$ et $\alpha_2$ sont des constantes inférieures à l'unité. Des valeurs typiques de $\alpha_1$ et $\alpha_2$ sont par exemple $\alpha_1 = \alpha_2 = 2/3$. D'autres valeurs, plus proches de zéro, pourraient permettre des recherches de points à énergie plus réduite, mais avec une phase de recherche plus longue, donc énergétiquement plus coûteuse.

En cas de perte de capture au point courant S (ce qui est le cas dans l'exemple de la Figure 5, puisque ce point S se trouve en deçà de la courbe de Lapicque CL pour le patient considéré (courbe qui définit la frontière entre les zones de capture ZC et de non-capture ZNC), on définit une fenêtre rectangulaire ADBC à partir des deux points A et B.

Le point B est choisi tel que :

$$\vec{B} = (\beta_1 t_c,\, \beta_2 V_c)$$

où $\beta_1$ et $\beta_2$ sont des constantes supérieures à l'unité.

Ce point B établit une valeur limite maximale d'énergie à tester dans la phase de recherche, énergie qui dépend donc, comme l'énergie minimale au point L, de la position du point courant S. Ce point B est déterminé de manière à correspondre à une énergie où l'on est sûr que la stimulation sera efficace, ce qui sera le cas si l'on prend par exemple pour $\beta_1$ et $\beta_2$ des valeurs $\beta_1 = 4$ et $\beta_2 = 2$.

Le point A est choisi comme étant le milieu du segment LB :

$$\vec{A} = \frac{\vec{L} + \vec{B}}{2}$$

On définit également le point M comme étant le centre du rectangle ADBC:

$$\vec{M} = \frac{\vec{A} + \vec{B}}{2}$$

Le point D du rectangle ADBC est alors le point tel que $t_D = t_A$, et $V_D = V_B$, et le point C est le point tel que $t_C = t_B$ et $V_C = V_A$ (le domaine ADBC étant un rectangle).

On définit alors quatre points de test pour mettre en oeuvre l'algorithme de recherche, à savoir les points A, M, C et D. Le point B sera considéré comme un "point de secours" : en cas de détection d'absence de capture, le dispositif appliquera immédiatement une contre-stimulation avec une énergie correspondant à celle du point B, pour compenser la perte de capture et pour être certain que l'impulsion de contre-stimulation sera une impulsion capturante.

La recherche du meilleur point parmi les quatre points de test A, M, C et D s'effectue dans l'ordre croissant de leur coût énergétique, avec itération de l'algorithme de recherche selon les étapes suivantes :

1) on définit le point de secours B' de la prochaine itération éventuelle de l'algorithme de recherche, qui sera le point B' = B ;

2) le point A est testé en premier, car il coûte moins en énergie que les autres points D, M ou C, la tension et/ou l'amplitude étant plus faibles en A qu'aux trois autres points. On applique donc une stimulation avec l'énergie correspondant à ce point A ;

3) si une capture est détectée lors du test au point A, on ne teste pas les points suivants D, M et C, et on définit un nouveau rectangle A'D'B'C' avec B' = A, de centre M' ;

4) en cas d'absence de capture lors du test au point A, on calcule des valeurs d'énergie proportionnelles aux énergies théoriques que coûtent les stimulations aux points D, M et C, selon la formule $Ep(i) = V(i)*V(i)*t(i)$, $i$ étant un point parmi D, M et C.

5) on classe les trois points D, M et C en fonction des valeurs $E_p(i)$ calculées à l'étape précédente, dans l'ordre décroissant, ce qui donne trois points X1, X2 et X3 tels que :

$$[\text{X1,X2, X3}]= \text{tri}(\{ \text{D,M,C}\}), \text{ avec } E_p(\text{X1}) < E_p(\text{X2}) < E_p(\text{X3})$$

6) le point X1 est alors testé. Si une capture est détectée, aucun test n'est effectué sur les points X2 et X3 et un nouveau rectangle est défini, avec B' = X1 ;

7) dans le cas contraire, une contre-stimulation est appliquée (point B) pour compenser la perte de capture, puis on teste le point X2 au cycle suivant ;

8) si une capture est détectée au point X2, aucun test n'est effectué sur le point X3 et un nouveau rectangle est défini, avec B' = X2 ;

9) dans le cas contraire, une contre-stimulation est appliquée (point B) pour compenser la perte de capture, puis on teste le point X3 au cycle suivant ;

10) si une capture est détectée au point X3, un nouveau rectangle est défini, avec B' = X3 ;

11) si une capture a été détectée à l'un des points X1, X2 ou X3, la procédure ci-dessus des étapes 1) à 10) est itérée, avec B = B' et A = (L+B')/2, c'est-à-dire que A est le milieu du segment LB' ;

12) si après réitération aucun des points de test n'a produit de capture, alors on met fin à l'algorithme de recherche et on définit comme valeur optimale d'énergie le dernier point B ayant produit la capture.

Dans une variante simplifiée, l'algorithme est arrêté après le premier point de test qui provoque une perte de capture. Le nombre d'étapes peut être ainsi réduit, avec toutefois un résultat présentant une moindre optimisation sur le plan énergétique.

Les Figures 6 et 7 sont des représentations du même type que celle de la Figure 5, appliquée à un premier exemple illustratif de mise en oeuvre de l'algorithme de la Figure 5 (sur la Figure 7 les courbes isoénergétiques ont été ajoutées à la représentation de la Figure 6).

Les points de test successifs sont numérotés dans l'ordre ① ... ⑨, et les points pour lesquels aucune capture n'a été détectée sont illustrés par des triangles.

On constate que, dans cet exemple, après neuf itérations l'algorithme a convergé vers le point ⑤ {0,8 V, 0,75 ms}, qui sera le point choisi comme optimum énergétique. Au cours de ces neuf itérations, cinq points n'ont pas permis de provoquer une capture (points n° ④, ⑥, ⑦, ⑧ et ⑨, et le point de secours (point ①) a été utilisé pour la contre-stimulation.

Sur la Figure 8 un autre exemple a été illustré, où dans ce cas l'algorithme converge après sept itérations, le point finalement retenu comme optimum énergétique étant le point ③ (dernier point ayant produit une capture). Dans la variante simplifiée de l'algorithme évoqué plus haut (qui consiste à mettre fin à l'algorithme dès le premier point qui ne génère pas de capture), l'algorithme prend fin après quatre itérations seulement, le point retenu comme optimum énergétique étant le point ③, c'est-à-dire dans ce cas précis (mais non nécessairement, de façon générale) le même point que dans la variante complète de l'algorithme.

## Revendications

**1.** Un dispositif médical implantable actif du type prothèse cardiaque de stimulation, resynchronisation et/ou défibrillation, comportant :

- un circuit de stimulation ventriculaire, apte à délivrer des impulsions de stimulation de faible énergie à une électrode implantable dans une cavité cardiaque d'un patient ;
- un circuit de test de capture, apte à détecter au cours d'un cycle cardiaque la présence ou l'absence d'une contraction consécutive à l'application d'une impulsion de stimulation ; et
- un circuit d'ajustement de l'énergie des impulsions délivrées par le circuit de stimulation, apte à contrôler indépendamment la tension de stimulation et la largeur de l'impulsion de stimulation,

**caractérisé en ce que** le circuit d'ajustement d'énergie comprend des moyens mettant en oeuvre un algorithme itératif de recherche d'optimum énergétique apte à modifier à la fois la largeur d'impulsion $t$ et la tension $V$ de chaque nouvelle impulsion délivrée, ces moyens étant aptes, à chaque itération courante, à effectuer les actions suivantes :

- définition d'une valeur $\{t, V\}$ d'énergie haute (B),
- définition d'une valeur $\{t', V'\}$ d'énergie basse (A), avec $t' < t$ et $V' < V$,
- délivrance d'une impulsion de stimulation avec la valeur d'énergie basse (A) puis test de capture, et

  e n présence d'une capture, fin de l'itération courante et passage à une nouvelle itération, avec l'énergie basse courante (A) comme nouvelle valeur d'énergie haute (B'),
  e n l'absence de capture, i) application d'une impulsion consécutive de contre-stimulation de largeur d'impulsion $t$ et de tension $V$ définies pour ladite valeur d'énergie haute, ii) achèvement de l'algorithme et iii) sélection de la dernière valeur d'énergie ayant produit la capture comme valeur d'optimum énergétique.

**2.** Le dispositif de la revendication 1, dans lequel les moyens mettant en oeuvre un algorithme itératif sont également aptes, à chaque itération courante, à effectuer les actions suivantes :

- définition d'une première valeur d'énergie intermédiaire $\{t', V\}$ (C),
- définition d'une deuxième valeur d'énergie intermédiaire $\{t, V'\}$ (D),
- définition d'une troisième valeur d'énergie intermédiaire $\{t'', V''\}$ (M), avec $t'<t''<t$ et $V'<V''<V$,
- classement des première, deuxième et troisième valeurs d'énergie intermédiaires (C, D, M) par valeur d'énergie décroissante,

et, en l'absence de capture après délivrance de l'impulsion avec la valeur d'énergie basse (A) puis test de capture :

- poursuite de l'itération courante avec délivrance d'impulsions de stimulation successivement avec les première, deuxième et troisième valeurs d'énergie intermédiaires (C, D, M) classées par valeur d'énergie décroissante, jusqu'à détecter une capture, et

  e n présence d'une capture, fin de l'itération courante et passage à une nouvelle itération, avec l'énergie intermédiaire courante ayant produit la capture comme nouvelle valeur d'énergie haute (B'), et
  e n l'absence de capture, achèvement de l'algorithme et sélection de la dernière valeur d'énergie ayant produit la capture, parmi les première, deuxième et troisième valeurs d'énergie intermédiaire, comme valeur d'optimum énergétique.

**3.** Le dispositif de la revendication 2, dans lequel ladite troisième valeur d'énergie intermédiaire (M) est une valeur $\{t'', V''\}$ telle que $t''=(t+t')/2$ et $V'' = (V+V')/2$.

**4.** Le dispositif de la revendication 1, dans lequel les valeurs d'énergie des impulsions délivrées par le circuit de stimulation sont au plus égales à une valeur limite maximale d'énergie, et dans lequel la valeur d'énergie haute $\{t,$

$V$} (B) de la première itération de l'algorithme est ladite valeur limite maximale d'énergie.

5. Le dispositif de la revendication 1, dans lequel les valeurs d'énergie des impulsions délivrées par le circuit de stimulation sont au moins égales à une valeur limite minimale d'énergie {$t_L$, $V_L$} (L), et dans lequel ladite valeur d'énergie basse (A) est une valeur {$t'$, $V'$} telle que $t' = (t+t_L)/2$ et $V' = (V+V_L)/2$.

6. Le dispositif de la revendication 1, dans lequel les valeurs d'énergie des impulsions délivrées par le circuit de stimulation sont comprises entre une valeur limite maximale d'énergie et une valeur limite minimale d'énergie calculées avant chaque première itération de l'algorithme.

7. Le dispositif de la revendication 6, dans lequel la largeur d'impulsion et la tension de la valeur limite maximale et de la valeur minimale d'énergie sont calculées par application de facteurs multiplicateurs, respectivement supérieur et inférieur à l'unité, à la largeur d'impulsion courante et à la tension courante du circuit de stimulation avant la première itération de l'algorithme.

**Patentansprüche**

1. Implantierbare aktive medizinische Vorrichtung des Typs Stimulations-, Resynchronisations- und/oder Defibrillations-Herzprothese, umfassend :

   - eine Ventrikel-Stimulations-Schaltung, die dazu geeignet ist, Stimulationsimpulse mit geringer Energie an eine in eine Herzkammer eines Patienten implantierbare Elektrode auszugeben;
   - eine Schaltung zur Prüfung einer erfolgreichen Stimulation, die dazu geeignet ist, in einem Herzzyklus das Vorhandensein oder die Abwesenheit einer Kontraktion in Folge des Anlegens eines Stimulationsimpulses zu detektieren, und
   - eine Schaltung zur Einstellung der Energie der durch die Stimulations-Schaltung ausgegebenen Impulse, die dazu geeignet ist, die Spannung der Stimulation und die Impulsbreite der Stimulation unabhängig voneinander zu steuern,

   **dadurch gekennzeichnet, dass** die Schaltung zur Einstellung der Energie Mittel umfasst, die einen iterativen Algorithmus zur Suche eines energetischen Optimums einsetzen, welcher dazu geeignet ist, sowohl die Impulsbreite $t$ und die Spannung $V$ jedes neu ausgegebenen Impulses zu verändern, wobei diese Mittel dazu geeignet sind, bei jeder aktuellen Iteration folgende Aktionen durchzuführen:

   - Festlegung eines hohen Energiewerts {$t$, $V$} (B),
   - Festlegung eines niedrigen Energiewerts {$t'$, $V'$} (A), wobei $t'<t$ und $V'<V$,
   - Ausgabe eines Stimulationsimpulses mit dem niedrigen Energiewert (A) und darauffolgende Prüfung einer erfolgreichen Stimulation, und

     • in Anwesenheit einer erfolgreichen Stimulation, Beendigung der aktuellen Iteration und Übergang zu einer neuen Iteration, mit der aktuellen niedrigen Energie (A) als neuen hohen Energiewert (B'),
     • in Abwesenheit einer erfolgreichen Stimulation, i) Anwendung eines konsekutiven Gegenstimulationsimpulses mit einer für den hohen Energiewert definierten Impulsbreite $t$ und Spannung $V$, ii) Beendigung des Algorithmus und iii) Wahl des letzten die erfolgreiche Stimulation bewirkenden Energiewerts als optimalen Energiewert.

2. Vorrichtung nach Anspruch 1, bei der die einen iterativen Algorithmus einsetzenden Mittel ebenfalls dazu geeignet sind, bei jeder aktuellen Iteration folgende Aktionen durchzuführen:

   - Festlegung eines ersten mittleren Energiewerts {$t'$, $V$} (C),
   - Festlegung eines zweiten mittleren Energiewerts {$t''$, $V''$} (M), wobei $t'<t''<t$ und $V'<V''<V$,
   - Ordnung der ersten, zweiten und dritten mittleren Energiewerte (C, D, M) in abnehmender Reihenfolge der Energiewerte,

   und in Abwesenheit einer erfolgreichen Stimulation nach der Ausgabe des Impulses mit dem niedrigen Energiewert (A) und anschließender Prüfung der erfolgreichen Stimulation:

- Weiterführung der aktuellen Iteration mit aufeinanderfolgender Ausgabe von Stimulationsimpulsen mit dem ersten, zweiten und dritten mittleren Energiewert (C, D, M) in abnehmender Reihenfolge der Energiewerte bis eine erfolgreiche Stimulation festgestellt wird, und

• in Anwesenheit einer erfolgreichen Stimulation, Beendigung der aktuellen Iteration und Übergang zu einer neuen Iteration, mit der aktuellen die erfolgreiche Stimulation bewirkenden mittleren Energie als neuen hohen Energiewert (B'), und
• in Abwesenheit einer erfolgreichen Stimulation, Beendigung des Algorithmus und Wahl des letzten die erfolgreiche Stimulation bewirkenden Energiewerts, unter den ersten, zweiten und dritten mittleren Energiewerten, als optimalen Energiewert.

3. Vorrichtung nach Anspruch 2, bei dem der dritte mittlere Energiewert (M) ein Wert $\{t'', V''\}$ ist, der folgende Gleichungen erfüllt: $t''=(t+t')/2$ und $V''=(V+V')/2$.

4. Vorrichtung nach Anspruch 1, bei der die Energiewerte der von der Stimulationsschaltung ausgegebenen Impulse höchstens gleich einem maximalen Energiegrenzwert sind, und bei der der hohe Energiewert $\{t, V\}$ (B) der ersten Iteration des Algorithmus dem maximalen Energiegrenzwert entspricht.

5. Vorrichtung nach Anspruch 1, bei der die Energiewerte der von der Stimulationsschaltung ausgegebenen Impulse mindestens gleich einem minimalen Energiegrenzwert $\{t_L, V_L\}$ (L) sind und bei der der niedrige Energiewert (A) ein Wert $\{t', V'\}$ ist, der folgende Gleichungen erfüllt: $t'=(t+t_L)/2$ und $V'=(V+VL)/2$.

6. Vorrichtung nach Anspruch 1, bei der die Energiewerte der von der Stimulationsschaltung ausgegebenen Impulse zwischen einem maximalen Energiegrenzwert und einem minimalen Energiegrenzwert liegt, welche vor jeder ersten Iteration des Algorithmus berechnet werden.

7. Vorrichtung nach Anspruch 6, bei der die Impulsbreite und die Spannung des maximalen Energiegrenzwerts und des minimalen Energiegrenzwerts durch Anwendung von Multiplikationsfaktoren, die jeweils grösser und kleiner als 1 sind, auf die aktuelle Impulsbreite und die aktuelle Spannung der Stimulationsschaltung vor der ersten Iteration des Algorithmus berechnet werden.

**Claims**

1. An active implantable medical device of the cardiac prosthesis type for stimulation, resynchronization and/or defibrillation, comprising:

- a ventricular stimulation circuit adapted to deliver low-energy stimulation pulses to an electrode that can be implanted into a heart cavity of a patient;
- a capture test circuit adapted to detect a presence or an absence of a contraction of the heart, during a cardiac cycle, subsequent to a stimulation pulse; and
- a circuit for adjusting the energy of the stimulation pulses delivered by the stimulation circuit, said adjustment circuit being adapted to independently control a stimulation voltage and a pulse width of the stimulation pulse ;

wherein the energy adjustment circuit comprises means which implement an iterative algorithm for searching an energetic optimum, which is adapted to modify both the pulse width $t$ and the voltage $V$ of each newly delivered pulse, wherein these means are adapted to implement the following operations at each current iteration:

- setting a value $\{t, V\}$ of high energy (B),
- setting a value $\{t', V'\}$ of low energy (A), with $t'<t$ and $V'<V$,
- delivering a stimulation pulse with the value of low energy (A) and performing a capture test, and

• in the presence of a capture, ending the current iteration and transitioning to a new iteration, wherein the value of low energy (A) is set as a new value of high energy (B'),
• in the absence of a capture, i) applying consecutive counter-stimulation pulses of a pulse width $t$ and a stimulation voltage $V$ set for the value of high energy, ii) ending the algorithm and iii) selecting the last energy value that produced the capture as an optimal energy value.

2. The device of claim 1, wherein the means implementing the iterative algorithm are also adapted to implement the following operations at each current iteration:

- defining a first intermediate energy value $\{t', V\}$ (C),
- defining a second intermediate energy value $\{t, V'\}$ (D)
- defining a third intermediate energy value $\{t'', V''\}$ (M), wherein $t'<t''<t$ and $V'<V''<V$,
- ranking the first, second, and third intermediate energy values (C, D, M) by decreasing energy value; and

in the absence of a capture after delivery of the pulse with the value of low energy (A) and subsequent capture test:

- continuing the current iteration with delivery of stimulation pulses in succession with the first, second and third intermediate energy values (C, D, M) ordered by decreasing energy value until detection of a capture; and

• in the presence of a capture, ending the current iteration and transitioning to a new iteration, wherein the current intermediate energy that produced the capture is set as a new high energy value (B') and
• in the absence of a contraction, ending the algorithm and selecting the last value of energy that produced the capture among the first, second and third intermediate energy values, as an optimal energy value.

3. The device according to claim 2, wherein said third intermediate energy value (M) is a value $\{t'', V''\}$ such that $t''=(t+t')/2$ and $V''=(V+V')/2$.

4. The device according to claim 1, wherein the energy values of the pulses delivered by the stimulation circuit are at most equal to a maximum energy limit value et wherein the high energy value $\{t, V\}$ (B) of the first iteration of the algorithm is said maximum energy limit value.

5. The device of claim 1, wherein the energy values of the stimulation pulses delivered by the stimulation circuit are at least equal to a lower energy limit value $\{t_L, V_L\}$ (L) and wherein said low energy value (A) is a value $\{t', V'\}$ such that $t'=(t+t_L)/2$ and $V'=(V+V_L)/2$.

6. The device of claim 1, wherein the energy values of the pulses delivered by the stimulation circuit are between an upper energy limit value and a lower energy limit value calculated before each first iteration of the algorithm.

7. The device of claim 6, wherein a pulse width and a voltage of the upper energy limit value and of the lower energy limit value are calculated by applying multiplication factors, respectively above and below 1, to a current pulse width and to a current voltage of the stimulation circuit before a first iteration of the algorithm.

Fig. 1

Fig. 2

Fig. 3

EP 3 042 693 B1

Fig. 4

Fig. 5

13

Fig. 6

Fig. 7

Fig. 8

**EP 3 042 693 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3777762 A
- US 4979507 A
- WO 9412237 A1
- US 5718720 A
- US 5702427 A
- US 5549652 A
- US 6650940 B1
- EP 2412401 A1
- EP 0552357 A1